**Europäisches Patentamt**

**European Patent Office**

**Office européen des brevets**

(19)

(11) Veröffentlichungsnummer : **0 466 638 A1**

(12) # EUROPÄISCHE PATENTANMELDUNG

(21) Anmeldenummer : **91810444.9**

(22) Anmeldetag : **12.06.91**

(51) Int. Cl.⁵ : **A61F 2/36**

(30) Priorität : **11.07.90 CH 2312/90**

(43) Veröffentlichungstag der Anmeldung :
**15.01.92 Patentblatt 92/03**

(84) Benannte Vertragsstaaten :
**AT CH DE FR GB IT LI**

(71) Anmelder : **GEBRÜDER SULZER
AKTIENGESELLSCHAFT
Zürcherstrasse 12
CH-8401 Winterthur (CH)**
Anmelder : **Protek AG
Stadtbachstrasse 64
CH-3001 Bern (CH)**

(72) Erfinder : **Cherubino, Paolo, Prof. Dr.-med.
Clinica Ortopedica dell'Università da Pavia
I-27100 Pavia (IT)**
Erfinder : **Pazzaglia, Ugo E., Prof. Dr.-med.
Clinica Ortopedica dell'Università da Pavia
I-27100 Pavia (IT)**
Erfinder : **Frick, Willi, Dr.
Eigenheimstrasse 11
CH-3084 Wabern (CH)**
Erfinder : **Koch, Rudolf
Oberdorfstrasse 229
CH-8267 Berlingen (CH)**

(54) **Femurkopfprothese.**

(57)  Die Erfindung betrifft eine Femurkopfprothese, die aus einem tragenden Schaftkörper (1) besteht, der proximal Oberflächenbereiche besitzt, die eine poröse, das Einwachsen von Knochengewebe fördernde Struktur (2) aufweisen. Erfindungsgemäss ist mit dem Schaftkörper (1) ein Drahtgeflecht (3) verbunden, welches von distal nach proximal durch Rippen (4) von innen abgestützt ist und welches Hohlräume (6) am Schaftkörper (1) überspannt, die von aussen über distale Oeffnungen (8) zwischen Drahtgeflecht (3) und Schaftkörper (1) und über Aussparungen (7) im Drahtgeflecht zugänglich sind, um Knochengewebe in die Hohlräume (6) einwachsen zu lassen und über einen Adernbaum zu versorgen.

EP 0 466 638 A1

Fig.3

Die Erfindung betrifft eine Femurkopfprothese bestehend aus einem tragenden Schaftkörper, der proximal Oberflächenbereiche besitzt, die eine poröse, das Einwachsen von Knochengewebe fördernde Struktur aufweisen.

Prothesenschäfte, die im proximalen Bereich mit Strukturen an ihrer Oberfläche versehen sind, die das Einwachsen des Schaftes fördern, sind vorbekannt. Die mittelfristige Verankerung geschieht dadurch, dass nach der Primärverankerung auf Vorsprüngen des Schaftes Knochengewebe von aussen in die Rücksprünge der Oberfläche einwächst. Bezogen auf die Schaftoberfläche entspricht dies einer einseitigen mechanischen Verankerung für die Uebertragung von Druckkräften.

Weiterhin sind hohle Schäfte in der Form von perforierten, selbsttragenden Rohrstücken bekannt, in die das Knochengewebe den ehemaligen Markraum füllend hineinwachsen soll. Das Verhältnis des im Schaft liegenden Knochenvolumens zu den für seine Blutversorgung vorgesehenen Oeffnungen in der Schaftwandung ist ungünstig. Zudem hat das im Schaft liegende Knochengewebe kaum Stützfunktion bei der Uebertragung von Kräften vom Schaft auf den Femurknochen, sondern eher die Funktion von Füllmasse.

Hier schafft die Erfindung Abhilfe. Sie löst die Aufgabe, die Schaftoberfläche auch von der Innenseite mit Knochengewebe zu verankern und eine ausreichende Blutversorgung auf der Innenseite für das Knochengewebe sicherzustellen. Diese Aufgabe wird dadurch gelöst, dass mit dem Schaftkörper ein Drahtgeflecht verbunden ist, welches von distal nach proximal durch Rippen von innen abgestützt ist und welches Hohlräume am Schaftkörper überspannt, die von aussen über distale Öffnungen zwischen Drahtgeflecht und Schaftkörper und über Aussparungen im Drahtgeflecht zugänglich sind, um Knochengewebe in die Hohlräume einwachsen zu lassen und über einen Adernbaum zu versorgen.

Die Vorteile der Erfindung sind darin zu sehen, dass über grössere Bereiche der Schaftoberfläche ein beidseitiges Einwachsen und ein Durchwachsen der Schaftoberfläche erfolgt, womit die Verankerung vom Schaft verbessert wird. Die fein verästelte, beidseitig wirkende Verankerung an einer begrenzt nachgiebigen Oberfläche ist auf Lastwechsel weniger empfindlich. Die abhängigen Ansprüche 2 und 3 beziehen sich auf vorteilhafte Weiterbildungen der Erfindung.

Im folgenden wird die Erfindung anhand von einem Ausführungsbeispiel beschrieben. Es zeigen:

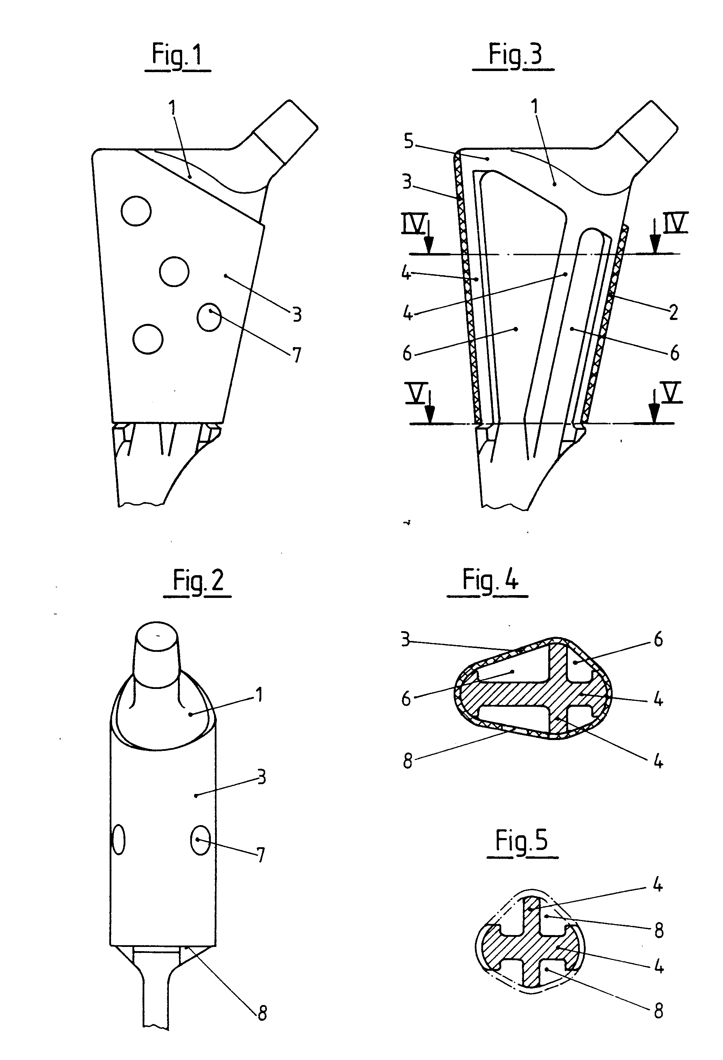

Fig. 1 den Ausschnitt einer Seitenansicht auf einen erfindungsgemässen Prothesenschaft;

Fig. 2 eine Ansicht des um 90° gedrehten Prothesenschaftes gemäss Fig. 1;

Fig. 3 einen teilweise geschnittenen Prothesenschaft gemäss Fig. 1;

Fig. 4, 5 Horizontalschnitte durch einen Prothesenschaft gemäss Fig. 3.

Die Figuren zeigen eine Femurkopfprothese, die aus einem tragenden Schaftkörper 1 besteht, der proximal Oberflächenbereiche besitzt, die eine poröse, das Einwachsen von Knochengewebe fördernde Struktur 2 aufweisen. Mit dem Schaftkörper 1 ist ein Drahtgeflecht 3 verbunden, welches von distal nach proximal durch Rippen 4 von innen abgestützt ist und welches Hohlräume 6 am Schaftkörper 1 überspannt. Die Hohlräume 6 sind von aussen über distale Oeffnungen 8 zwischen Drahtgeflecht 3 und Schaftkörper 1 und über Aussparungen 7 im Drahtgeflecht 3 zugänglich, um Knochengewebe in die Hohlräume 6 einwachsen zu lassen und über einen Adernbaum zu versorgen.

Der Schaftkörper 1 bildet im proximalen Bereich mit den Rippen 4, die teilweise in Wülste auslaufen, einen Trägerkörper mit Hohlräumen 6, über die das Drahtgeflecht 3 gespannt ist. Proximal schliesst das Drahtgeflecht 3 mit einer Querrippe 5 ab, während gegen distal Oeffnungen 8 bestehen, durch die ein Adernbaum einwachsen kann. Aussparungen 7 im Drahtgeflecht 3 sollen ebenfalls mit besserer Blutversorgung das Einwachsen des Knochengewebes fördern, ohne zuviel an Stützfläche zu verlieren.

Die Primärverankerung vom Schaft erfolgt im Schaftunterteil und proximal im Bereich der vorstehenden Rippen 4, 5. Durch das beidseitig vom Drahtgeflecht 3 einwachsende Knochengewebe und durch das teilweise Durchwachsen des Drahtgeflechts entsteht eine intensive Verankerung. Mit seiner begrenzten Nachgiebigkeit im Bereich der überspannten Hohlräume 6 reduziert das Drahtgeflecht Spannungen, die sonst als Spitzen am verankerten Knochengewebe auftreten würden. Das Einwachsen des Knochengewebes in die von Drahtgeflecht überspannten Hohlräume 6 wird durch Beschichten der Wandungen des Schaftkörpers 1 im Bereich der Rippen 4 mit Hydroxylapatit zusätzlich gefördert.

## Patentansprüche

1. Femurkopfprothese bestehend aus einem tragenden Schaftkörper (1), der proximal Oberflächenbereiche besitzt, die eine poröse, das Einwachsen von Knochengewebe fördernde Struktur (2) aufweisen, dadurch gekennzeichnet, dass mit dem Schaftkörper (1) ein Drahtgeflecht (3) verbunden ist, welches von distal nach proximal durch Rippen (4) von innen abgestützt ist und welches Hohlräume (6) am Schaftkörper (1) überspannt, die von aussen über distale Öffnungen (8) zwischen Drahtgeflecht (3) und Schaftkörper (1)

und über Aussparungen (7) im Drahtgeflecht (3) zugänglich sind, um Knochengewebe in die Hohlräume (6) einwachsen zu lassen und über einen Adernbaum zu versorgen.

2. Femurkopfprothese nach Anspruch 1, dadurch gekennzeichnet, dass das Drahtgeflecht (3) elastisch federnd ausgeführt ist, um die Steifigkeit des Drahtgeflechtes, welches Hohlräume (6) überspannt, den zulässigen Spannungen im umgebenden Knochengewebe anzupassen.

3. Femurkopfprothese nach Anspruch 2, dadurch gekennzeichnet, dass die feste Wandung der mit Drahtgeflecht (3) überspannten Hohlräume (6) mit einer das Knochenwachstum fördernden Schicht zum Beispiel aus Hydroxylapatit überzogen ist.

Fig.1

Fig.3

Fig.2

Fig.4

Fig.5

Europäisches
Patentamt

**EUROPÄISCHER RECHERCHENBERICHT**

Nummer der Anmeldung

EP 91 81 0444

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 315 902 (CERAVER) <br> * Seite 2, Zeilen 12-31; Figuren * | 1,2 | A 61 F 2/36 |
| Y | | 3 | |
| | --- | | |
| Y | EP-A-0 340 174 (CREMASCOLI) <br> * Anspruch 3 * | 3 | |
| | --- | | |
| A | EP-A-0 071 242 (CERAVER) <br> * Zusammenfassung; Figuren * | 1,2 | |
| | --- | | |
| A | EP-A-0 093 378 (WALDEMAR LINK) <br> * Seite 11, Zeile 6 - Seite 12, Zeile 10; Figuren 1-4 * | 1 | |
| | --- | | |
| A | EP-A-0 230 006 (WALDEMAR LINK) | | |
| | --- | | |
| A | DE-A-2 758 541 (3M) | | |
| | --- | | |
| A | FR-A-2 519 248 (TIMOTEO) | | |
| | ----- | | |

| | | | RECHERCHIERTE SACHGEBIETE (Int. Cl.5) |
|---|---|---|---|
| | | | A 61 F |

Der vorliegende Recherchenbericht wurde für alle Patentansprüche erstellt

| Recherchenort | Abschlußdatum der Recherche | Prüfer |
|---|---|---|
| DEN HAAG | 25-09-1991 | KLEIN C. |

EPO FORM 1503 03.82 (P0403)